Europäisches Patentamt

European Patent Office

Office européen des brevets

(11) Numéro de publication: **0 227 553 B1**

## (12) FASCICULE DE BREVET EUROPEEN

(45) Date de publication de fascicule du brevet: **11.03.92**  (51) Int. Cl.5: **C08J 5/18**, A61L 15/16

(21) Numéro de dépôt: **86402870.9**

(22) Date de dépôt: **19.12.86**

(54) **Procédé de préparation d'un film souple d'alginate de calcium.**

(30) Priorité: **26.12.85 FR 8519239**

(43) Date de publication de la demande:
**01.07.87 Bulletin 87/27**

(45) Mention de la délivrance du brevet:
**11.03.92 Bulletin 92/11**

(84) Etats contractants désignés:
**CH DE ES FR GB IT LI**

(56) Documents cités:
**EP-A- 0 044 624**
**DE-A- 1 470 890**
**GB-A- 621 230**
**GB-A- 653 341**

(73) Titulaire: **Société PRECIS**
**43 rue de Neuilly**
**F-92000 Nanterre Hauts-de-Seine(FR)**

(72) Inventeur: **Adeline, André**
**18, avenue du Lac Domaine de Croix-Marie**
**F-78121 Crespières(FR)**

(74) Mandataire: **Bloch, Gérard et al**
**2, square de l'Avenue du Bois**
**F-75116 Paris(FR)**

## Description

L'alginate de calcium possède des propriétés bien connues comme hémostatique et cicatrisant et, présenté sous forme de fibres, permet le cheminement des fibroplastes et la régénération des tissus lésés. Il présente toutefois l'inconvénient d'avoir une structure tridimensionnelle mal définie et une solubilité dans l'eau insuffisante conduisant à une mauvaise résorption.

L'invention a pour but de présenter l'alginate de calcium sous forme de film souple, d'épaisseur variable à volonté, ayant une structure poreuse et alvéolaire et une solubilité dans les liquides biologiques adaptable selon les besoins.

A cet effet, l'invention a pour objet un procédé de préparation d'un film souple d'alginate de calcium, caractérisé par le fait qu'on mélange une solution d'alginate de sodium avec une solution de chlorure de calcium sous agitation contrôlée, en présence d'un gaz inerte, on élimine le chlorure de sodium formé par ionophorèse (ou électrodialyse), on congèle rapidement l'émulsion produite, puis on lyophilise l'émulsion.

Dans la première phase du procédé, à savoir l'incorporation d'un gaz dans la solution naissante d'alginate de calcium, produite par le mélange d'alginate de sodium et d'alginate de calcium, le gaz peut être généré par un liquide, par exemple du chlorofluoréthane, sous l'effet d'une variation de pression ou température.

Le procédé de l'invention est décrit avec plus de détails dans ce qui suit, sans pour cela le limiter à cette description d'un mode préférentiel de mise en oeuvre.

Dans une chambre cylindrique, dans laquelle tourne une hélice d'agitateur, on introduit, par trois arrivées distinctes, respectivement, une solution d'alginate de sodium, une solution de chlorure de calcium et de l'azote, étant entendu que l'azote peut être remplacé par un autre gaz inerte ou un liquide générateur de gaz, tel que le fréon®.

Lors du mélange sous forte agitation de la solution d'alginate de sodium et de la solution de chlorure de calcium il y a augmentation brusque de la viscosité en même temps qu'incorporation du gaz sous forme de microbulles.

Les caractéristiques opératoires sont :

- solution d'alginate de sodium à 2,5 % et de viscosité comprise entre 100 et 300 mPa•s à 18°C ;
- solution de chlorure de calcium à 0,25 % de calcium.

En faisant varier le débit de la solution de calcium, on obtient un alginate comprenant entre 0,30 % de calcium et 1,20 % de calcium et tout l'éventail de solubilité dans l'eau et les liquides organiques.

C'est ainsi que l'alginate à 0,30 % de calcium est très soluble, alors que l'alginate à 1,20 % est insoluble.

L'excès de ClNa générée est éliminée par ionophorèse (ou électrodialyse).

La solution pâteuse, obtenue après mélange, est refroidie, entre 0 et 10°C environ, et passe entre des plaques de membranes échangeuses d'ions, écartées l'une de l'autre de 5 à 10 mm. De part et d'autre de ces plaques, sont ménagés des compartiments munis d'électrodes et dans lesquels circule de l'eau refroidie. Les électrodes sont reliées à une source de courant continu à potentiel variable. La solution d'alginate de calcium circule sous pression et des électrodes sélectives $Cl^-$ et $Na^+$ situées à la sortie permettent de connaître la teneur résiduelle en ClNa donc de controler celle-ci par variation de la vitesse d'écoulement ainsi que de la différence de potentiel entre les compartiments anodique et cathodique.

Le calcium de l'alginate de Ca complexé a une mobilité électrophorètique de beaucoup inférieure à celle de Cl et Na ce qui permet d'éliminer ces deux ions partiellement ou en totalité.

La solution obtenue est répartie en couches d'épaisseur comprise entre 2 et 10 mm, l'optimum se situant entre 3 et 6 mm selon la destination du produit.

Après congélation rapide à basse température de l'émulsion congelée et éventuellement dégazage, elle est lyophilisée.

On obtient une plaque souple, blanc crème , ayant une structure fibrillaire et microporeuse, à pores ouverts, et plus ou moins soluble dans l'eau, suivant le rapport Ca/Na de sa constitution.

Cette plaque, suivant sa teneur relative en calcium et sodium, peut servir d'hémostatique et de cicatrisant, ainsi que de support de cellules en culture.

## Revendications

1. Procédé de préparation d'un film souple d'alginate de calcium, caractérisé par le fait qu'on mélange une solution d'alginate de sodium avec une solution de chlorure de calcium sous agitation contrôlée, en présence d'un gaz inerte, on élimine le chlorure de sodium formé, on congèle rapidement l'émulsion produite, puis on lyophilise l'émulsion.

2. Procédé selon la revendication 1, dans lequel on élimine le chlorure de sodium par électrodialyse.

3. Procédé selon la revendication 1, dans lequel le gaz incorporé dans la solution naissante d'alginate de calcium est générée par un liquide sous l'effet d'une variation de pression ou de température.

4. Procédé selon la revendication 3, dans lequel le liquide générateur de gaz est le chlorofluoréthane.

## Claims

1. A method of preparing a flexible calcium alginate film, characterized in that a sodium alginate solution is mixed with a calcium chloride solution under controlled agitation, in the presence of an inert gas, the sodium chloride formed is eliminated, the emulsion produced is rapidly congealed then the emulsion is liophylized.

2. The method as claimed in claim 1, wherein the sodium chloride is eliminated by electrodialysis.

3. The method as claimed in claim 1, wherein the gas incorporated in the nascent calcium alginate solution is generated by a liquid under the effect of a pressure or temperature variation.

4. The method as claimed in claim 3, wherein the gas generating liquid is chlorofluorethane.

## Patentansprüche

1. Verfahren zur Herstellung einer biegsamen Calciumalginatfolie, dadurch gekennzeichnet, daß eine Natriumalginatlösung unter Kontrollierter Bewegung mit einer Kalziumchloridlösung bei Vorhandensein von Schutzgas gemischt wird, das sich bildende Natriumchlorid eliminiert, die erzeugte Emulsion rasch gefroren und die Emulsion anschließend lyophilisiert wird.

2. Verfahren gamäß Anspruch 1, bei welchem das Natriumchlorid durch Elektrodialyse eliminiert wird.

3. Verfahren gamäß Anspruch 1, bei welchem das in die entstehende Calciumalginatlösung eingemischte Gas durch eine Flüssigkeit unter Einwirkung einer Druck - order Temperaturveränderung erzeugt wird.

4. Verfahren gamäß Anspruch 3, bei welchem die gaserzeugende Flüssigkeit das Chlorfluorethan ist.